# EUROPEAN PATENT APPLICATION

(11) **EP 3 106 513 A1**
(43) Date of publication of application: **21.12.2016**
(21) Application number: 15749199.4
(22) Date of filing: 12.02.2015
(51) Int. Cl.: C12N 1/00, C02F 3/34, C08J 11/10, C12N 1/20

(54) **METHOD FOR DECOMPOSING POLYHYDROXYALKANOIC ACID, AND MICROORGANISM PREPARATION**

(30) Priority: 14.02.2014 JP 2014026498
(71) Applicant: Kaneka Corporation, Osaka-shi, Osaka 530-8288 (JP); Niigata University Of Pharmacy And Applied Life Sciences, Niigata-shi, Niigata 956-0841 (JP)
(72) Inventor: SHIGEMATSU, Toru, Niigata 956-0841 (JP); IGUCHI, Akinori, Niigata 956-0841 (JP); SUGAYA, Takahiko, Osaka 566-0072 (JP); MATSUMOTO, Keiji, Osaka 530-8288 (JP)
(74) Representative: Vossius & Partner Patentanwälte Rechtsanwälte mbB
(86) International application number: PCT/JP2015/000644
(87) International publication number: WO 2015/122190

(57) **Abstract**

Provided are methods for decomposing a polyhydroxyalkanoic acid using an anaerobic microbial community for decomposing a polyhydroxyalkanoic acid efficiently to convert the polyhydroxyalkanoic acid to a biogas including methane as a main component; and a microbial preparation usable as seed sludge for a methane fermentation process. The polyhydroxyalkanoic acid is subjected to decomposing treatment with a microbial community including a methane-producing archaeon in the following (A), and a bacterium in the following (B): (A) a methane-producing archaeon belonging to the genus Methanosarcina, or a single-rod-shaped hydrogen-utilizing methane-producing archaeon; and (B) a bacterium which is classified into any one of the phylum Acidobacteria, the phylum Bacteroidetes, the phylum Chloroflexi, the phylum Firmicutes, the phylum Nitrospirae, the phylum Proteobacteria, the phylum Synergistetes, the phylum Thermotogae and an unclassified phylum, and is capable of decomposing the polyhydroxyalkanoic acid.

## Description

### Technical Field

The present invention relates to a method for decomposing a polyhydroxyalkanoic acid, and a microorganism preparation used for the decomposition.

### Background Art

In recent years, technical development has been advanced for converting organic waste materials, such as kitchen wastes discharged from households, school lunches and restaurants to a biogas including methane as a main component by a methane fermentation method. The biogas obtained by this technique can be reused as energy. Thus, costs for waste disposal can be decreased, and further an energy-producing industry can be created.

However, in order to use organic waste materials for a methane fermentation process, it is necessary to separate and remove plastic wastes contained in the materials. For such separation and removal, a large quantity of labor is required, and thus this matter is a large problem against methane fermentation technique.

So far, a number of microorganisms have reportedly been demonstrated to accumulate, in their body cells, polyester as an energy storage substance. A polyhydroxyalkanoic acid (hereinafter may be referred to as a PHA), which is a homopolymer or copolymer of a 3-hydroxyalkanoic acid, is a thermoplastic polymer. Since PHA can be decomposed by microorganisms in composting processes, or in the natural environment, a significant attention has been paid to PHA as an environment-friendly plastic material. For this reason, development of such biodegradable plastic materials, including PHAs, have been advanced for the application of biodegradable plastic materials to materials for agriculture that are used in the environment, food containers that are not easily recovered or reused after use, packaging materials, sanitary materials, garbage bags and others. The above-mentioned problem will be solved, particularly, when it is unnecessary to separate and remove plastic wastes when food containers, packaging materials, garbage bags and others are used to methane fermentation. Thus, a large expectation is given to the development.

However, due to the high amount of plastic materials currently used, it is not likely that all biodegradable plastic materials used could be treated only by the decomposition by microorganisms present in the natural environments or any fermentation process. Thus, methods have been investigated to screen and isolate PHAs decomposing microorganisms and to use them for decomposition of the PHAs positively.

PHAs are classified into short-chain-length PHAs (hereinafter may be referred to as PHASCLs, which consist of alkanes with 3 to 5 carbon atoms), and medium-chain-length PHAs (hereinafter abbreviated as PHAMCLs, which consist of alkanes with 6 to 14 carbon atoms). Microbial strains that decompose a PHA have been demonstrated to widely be distributed in bacteria and funguses, and a large number of strains that are able to decompose a PHASCL or PHAMCL have been isolated (Non-Patent Literature 1). Most of bacterial strains can specifically decompose PHASCLs or PHAMCLs. As a bacterial species, which can decompose both types of PHAs, Streptomyces exfoliatus has been reported. This species has been demonstrated to have two or more degrading enzymes which correspond to the two types of PHAs, respectively, so that it can decompose both types of PHAs (Non-Patent Literature 2, and Non-Patent Literature 3).

Recently, researches have been conducted on a copolymer polyester (hereinafter may be abbreviated as a PHBH) of 3-hydroxybutyric acid (hereinafter may be abbreviated as 3HB), as a kind of PHASCL, and 3-hydroxyhexanoic acid (hereinafter may be abbreviated as 3HH), as a kind of PHAMCL, and on manufacturing method of PHBH.

Although only limited numbers of researches on degrading enzymes for PHBHs has been reported, a PHB degrading enzyme from a Bacillus megaterium N18-25-9 strain and its recombinant enzyme, which is generated by the transformant harboring the gene encoding this PHBH degrading enzyme isolated from this strain and a PHB enzyme gene expression vector (Non-Patent Literature 3, and Non-Patent Literature 4). The growth temperature of this strain ranges from 20 to 45°C.

Moreover, Thermobifida fusca, which decomposes PHBHs, has been isolated. The depolymerase gene was cloned from this bacteria and introduced into host cells. The activity of the resulting recombinant PHBHs depolymerase expressed in the host cells showed that the optimal temperatures were as high as from 65 to 75°C, and that the PHBH decomposing activity thereof was remarkably high (Patent Literature 1).

However, all of these excellent researches are on the decomposition of PHAs by microorganisms growing in aerobic environments. Methane fermentation is a process, which converts organic matters to methane by interaction within anaerobic microbial communities. Thus, in order to apply methane fermentation on the treatment of PHAs including PHBHs or organic wastes including PHBHs, it is necessary to use an anaerobic microbial community having a capability of decomposing PHAs including PHBHs.

However, researches on the decomposition of PHAs by methane fermentation have hardly been known so far.

### Citation List

### Patent Literature

PTL 1: JP 2009-207424 A

### Non-Patent Literatures

Non-Patent Literature 1: Jendrossek et al., Ann. Rev. Microbiol. 2002, vol. 56,403.
Non-Patent Literature 2: Schirmer et al., Appl. Environ. Microbiol., vol. 59,1220 (1993)
Non-Patent Literature 3: Klingbeil et al., FEMS Microbiol. Lett., vol. 142,215 (1996)
Non-Patent Literature 4: Takaku et al., FEMS Microbiol. Lett., vol. 264,152 (2006)

### Summary of Invention

### Technical Problem

In light of the above-mentioned situation at present, an object of the present invention is to provide a method for decomposing a PHA, using an anaerobic microbial community for decomposing the PHA efficiently to be converted to a biogas including methane as a main component, and a microbial preparation, which can be used as a seed sludge for a methane fermentation process.

### Solution to Problem

In order to solve the above-mentioned problems, the present inventors have repeated researches eagerly to find out the following: the inventors carried out anaerobic continuous cultivation of an anaerobic digester tank sludge as a seed sludge by supplying with a synthetic wastewater containing a PHA powder and an acetate as carbon sources. The resulting acclimatized sludge with a specific microbial community showed an efficient decomposing performance of PHA and production of a biogas containing methane as a main component. Thus, the present invention has been accomplished.

Accordingly, the present invention provides the following items (1) to (11).
(1) A method for biologically decomposing a polyhydroxyalkanoic acid, the method comprising subjecting a polyhydroxyalkanoic acid to decomposing treatment with a microbial community comprising a methane-producing archaeon in (A) below and a bacterium in (B) below:
   (A) a methane-producing archaeon belonging to the genus Methanosarcina, or a single-rod-shaped hydrogen-utilizing methane-producing archaeon, and
   (B) a bacterium which is classified into any one of the phylum Acidobacteria, the phylum Bacteroidetes, the phylum Chloroflexi, the phylum Firmicutes, the phylum Nitrospirae, the phylum Proteobacteria, the phylum Synergistetes, the phylum Thermotogae, and an unclassified phylum, and is capable of decomposing the polyhydroxyalkanoic acid.
(2) The method for decomposing a polyhydroxyalkanoic acid, wherein the archaeon in (A) comprises the methane-producing archaeon belonging to the genus Methanosarcina.
(3) The method for decomposing a polyhydroxyalkanoic acid, wherein the archaeon in (A) comprises both of the methane-producing archaeon belonging to the genus Methanosarcina and the single-rod-shaped hydrogen-utilizing methane-producing archaeon.
(4) The method for decomposing a polyhydroxyalkanoic acid, wherein the bacterium in (B) comprises a bacterium classified into the phylum Firmicutes.
(5) The method for decomposing a polyhydroxyalkanoic acid, wherein a proportion of the bacterium classified into the phylum Firmicutes in a whole of the microbial community is from 50 to 95% both inclusive in terms of a proportion of clones of a 16S rRNA gene.
(6) The method for decomposing a polyhydroxyalkanoic acid, wherein the bacterium classified into the phylum Firmicutes comprises a bacterium classified into the family Peptococcaceae or the family Thermoanearobacteraceae in a total proportion of 20 to 95% both inclusive in terms of the proportion of the clones of the 16S rRNA gene.
(7) The method for decomposing a polyhydroxyalkanoic acid, wherein the polyhydroxyalkanoic acid is subjected to the decomposing treatment under an anaerobic condition.
(8) The method for decomposing a polyhydroxyalkanoic acid, wherein the polyhydroxyalkanoic acid is subjected to the decomposing treatment under a condition of a temperature from 20 to 65°C.
(9) The method for decomposing a polyhydroxyalkanoic acid, wherein the polyhydroxyalkanoic acid is a polymer comprising, as a recurring unit structure, at least one of 3-hydroxybutyric acid and 3-hydroxyhexanoic acid.
(10) The method for decomposing a polyhydroxyalkanoic acid, wherein the polyhydroxyalkanoic acid is a polymer comprising, as recurring unit structures, 3-hydroxybutyric acid and 3-hydroxyhexanoic acid.
(11) A microbial preparation for decomposing a polyhydroxyalkanoic acid and producing methane, the preparation comprising a methane-producing archaeon in (A) below and a bacterium in (B) below:
   (A) a methane-producing archaeon belonging to the genus Methanosarcina, or a single-rod-shaped hydrogen-utilizing methane-producing archaeon, and
   (B) a bacterium which is classified into any one of the phylum Acidobacteria, the phylum Bacteroidetes, the phylum Chloroflexi, the phylum Firmicutes, the phylum Nitrospirae, the phylum Proteobacteria, the phylum Synergistetes, the phylum Thermotogae and an unclassified phylum, and is capable of decomposing the polyhydroxyalkanoic acid.

### Advantageous Effects of Invention

The present invention makes it possible to decompose a PHA such as PHBH efficiently and inexpensively to produce a biogas containing methane as a main component. In this way, kitchen wastes can be disposed of in the state of being put in a garbage bag made of PHA such as PHBH to be reused as energy.

### Brief Description of Drawings

Fig. 1(a) is a phase-contrast photomicrograph image of microbial communities in the reactor supplied with a synthetic wastewater containing a PHBH and an acetate in carbon concentration ratio of 2/8 at a dilution ratio of 0.05 d⁻¹.
Fig. 1(b) is a fluorescence photomicrograph image.
Fig. 2 is a phylogenetic tree generated by 16S rRNA gene clones detected in the reactor in the the phylum Firmicutes.

### Description of Embodiments

Hereinafter, the present invention will be described in detail.

The present invention provides a method for efficiently decomposing a PHA, wherein a specific microbial community is used to biologically decompose the PHA, particularly, under an anaerobic condition, so that the PHA is converted to a biogas containing methane as a main component.

The PHA in the present invention is represented by the following general formula.

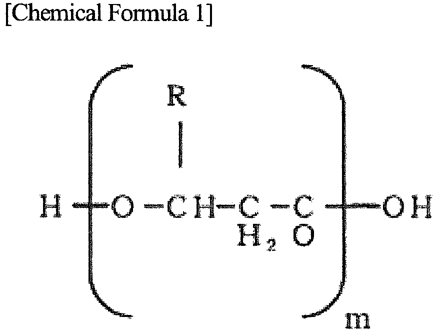

In the formula, Rs each represent an alkyl group having 1 to 13 carbon atoms, and m represents an integer of 2 or more, and Rs, the number of which is m, may be the same or different.

In the present invention, the PHA is preferably a PHA of at least one of 3HB and 3HH, and is more preferably a copolymer of 3HB and 3HH, and represented by the following general formula.

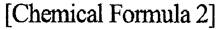

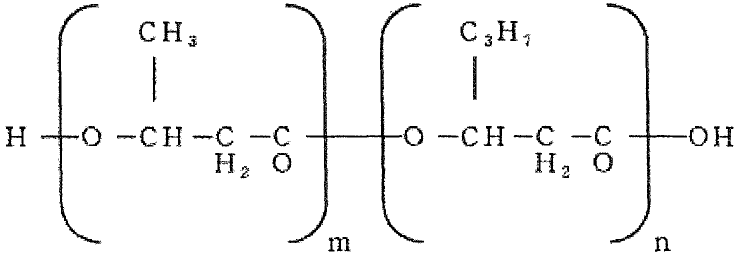

In the formula, m and n each represent an integer of 1 or more.

The microbial community in the present invention is composed of a methane-producing archaeon (A); and a microorganism (bacterium) (B) taking charge of a process of decomposing a PHA, particularly, a PHBH, and producing substances that are to be substrates for the methane-producing archaeon, such as acetic acid, hydrogen, and carbon dioxide.

For further details, the methane-producing archaeon (A) is a methane-producing archaeon belonging to the genus Methanosarcina, or a single-rod-shaped hydrogen-utilizing methane-producing archaeon. It is preferred from the viewpoint of PHA decomposing performance that the methane-producing archaeon (A) includes a methane-producing archaeon belonging to the genus Methanosarcina. It is more preferred from the viewpoint of the better PHA decomposing performance that the methane-producing archaeon (A) includes both of a methane-producing archaeon belonging to the genus Methanosarcina and a single-rod-shaped hydrogen-utilizing methane-producing archaeon.

The bacterium (B) is not particularly limited as far as the bacterium is classified into any one of the phylum Acidobacteria, the phylum Bacteroidetes, the phylum Chloroflexi, the phylum Firmicutes, the phylum Nitrospirae, the phylum Proteobacteria, the phylum Synergistetes, the phylum Thermotogae and an unclassified phylum, and is capable of decomposing a PHA. The bacterium (B) preferably includes a bacterium classified into the phylum Firmicutes since the bacterium is excellent in PHA decomposing performance. For the proportion of the bacterium classified, particularly, in the phylum Firmicutes in the whole of the microbial community, the lower value of the proportion is preferably 50% or more, more preferably 60% or more, in particular preferably 69% or more, in terms of the proportion of clones of a 16S rRNA gene. The upper value of the proportion is preferably 95% or less, more preferably 85% or less, in particular preferably 79% or less.

From the viewpoint of PHA decomposing performance, it is also preferred that the bacterium classified into the phylum Firmicutes includes a bacterium classified into the family Peptococcaceae or the family Thermoanearobacteraceae; and further it is preferred that for the total proportion of the bacterium classified into the family Peptococcaceae or the family Thermoanearobacteraceae in the bacteria classified into the phylum Firmicutes, the lower value of the proportion is preferably 20% or more, more preferably 25% or more, in particular preferably 29% or more, in terms of the proportion of the clones of the 16S rRNA gene. The upper value of the proportion is preferably 95% or less, more preferably 90% or less, in particular preferably 83% or less.

The anaerobic microbial community in the present invention has an activity for decomposing a PHA, examples thereof including a PHB and a PHBH.

The decomposing treatment temperature when a PHA is decomposed by the present invention is preferably from 20 to 65°C, more preferably from 25 to 50°C, in particular preferably from 30 to 40°C since the microorganism activity is made excellent.

In an ordinary methane fermentation process, the process is operated under either a condition of temperatures from 30 to 40°C, or a condition of temperatures from 50 to 65°C.

The method for decomposition of the present invention is very useful since the method is applicable to the existing facilities when a PHA is decomposed to be converted into a biogas in, for example, a disposal facility having methane fermentation equipment.

The microbial community used in the present invention is also characterized in that the decomposing activity thereof is improved by the coexistence of t acetic acid and hydrolyzates of proteins.

The decomposing method of the present invention is characterized in that a PHA can be efficiently decomposed under an anaerobic condition, and is very useful.

The microbial community in the present invention can be made into a microbial preparation usable in a methane fermentation process for the purpose of decomposing a PHA. This microbial preparation is usable as seed sludge in a methane fermentation process.

By using the microorganism preparation to construct a methane fermentation process, a biogas containing methane as a main component can be obtained by using, as raw material, organic wastes containing a PHA.

### (Method for Enriching PHA-Decomposable Anaerobic Microbial Community)

The microbial community in the present invention can be enriched from sludge in a methane fermentation process, such as sludge in an existing anaerobic digestion tank. However, the method for the enrichment is not particularly limited as far as the method makes it possible to enrich a microorganism group capable of decomposing a PHA efficiently.

The following will describe, as the enrichment method, a method of enrichment of a microorganism group through a methane fermentation process of using sludge as seed sludge, and continuously supplying a synthetic wastewater containing a PHBH and an acetate as carbon sources. However, the present invention is not limited to this method.

An example of the preferable enrichment method can include a method of using a completely stirred tank reactor to perform a continuous culture in a chemostat-manner by supplying the reactor continuously with a synthetic wastewater containing an emulsified PHBH and an acetate as carbon sources, and further discharging an in-tank liquid at a rate equal to the supplying rate of the synthetic wastewater.

The ratio in concentration of the PHBH and the acetate in the synthetic waste water is not particularly limited as far as this concentration ratio in terms of carbon concentration permits a target microorganism group to be enriched. It is preferred to use a synthetic wastewater in which the ratio is from 1/9 to 5/5, particularly, 1/9 to 3/7 since the ratio easily gives a microbial community having PHA decomposing performance.

The seed sludge is not particularly limited as far as the sludge makes it possible to enrich the microbial community used in the present invention. It is preferred to use an in-tank liquid for a methane fermentation process for disposing of kitchen wastes since an efficient enrichment can be attained.

The organic carbon concentration in the synthetic waste liquid is preferably from 5,000 mg/L to 10,000 mg/L, in particular preferably from 7,000 mg/L to 9,000 mg/L since a microorganism group having PHA decomposing performance is easily obtained.

The dilution ratio of the synthetic waste liquid is preferably from 0.01 to 0.1 d⁻¹, in particular preferably from 0.025 to 0.05 d⁻¹ (hydraulic residence time: 20 to 40 days) since a microorganism group having PHA decomposing performance is easily obtained.

When the operating temperature ranges from 25 to 50°C, no problem is caused. It is preferred to conduct the operation at 30 to 40°C since a microorganism group high in PHA decomposing performance is easily obtained.

The enrichment is performed preferably under an anaerobic condition.

When the operation is performed over a period two or three times of the hydraulic residence time, the system reaches to a steady state to complete the enrichment. Thus, in the in-tank liquid of the reactor, a microbial community used in the present invention is enriched.

The microbial community enriched by the above-mentioned method has performance of decomposing a PHA efficiently to produce methane.

### (Method for Decomposing PHA)

The following will describe a method for decomposing a PHA, using the enriched microbial community.

The enriched microbial community includes the following microorganisms: (A) a methane-producing archaeon belonging to the genus Methanosarcina, or a single-rod-shaped hydrogen-utilizing methane-producing archaeon; and (B) a bacterium which is classified into any one of the phyla Acidobacteria, Bacteroidetes, Chloroflexi, Firmicutes, Nitrospirae, Proteobacteria, Synergistetes and Thermotogae, and an unclassified phylum, and which is capable of decomposing a PHA.

The decomposition of the PHA can be attained, for example, by anaerobic batch culture using a vial.

An mineral salt medium composed of potassium dihydrogenphosphate, potassium hydrogen carbonate and some other, and a PHA in a sheet or some other form are put into a glass vial, and the vial is airtightly closed with, for example, a butyl rubber stopper. Thereafter, while the airtight sealed state is kept, an in-tank liquid of a reactor, which contains a microbial community, is added to the vial. In this way, batch culture can be attained while the vial is shaken.

The temperature during the culture is preferably from 20 to 65°C, more preferably from 25 to 50°C, in particular preferably from 30 to 40°C.

The reaction is conducted preferably under an anaerobic condition since this condition makes the decomposing performance of the microbial community excellent. Thus, before the airtight sealing, sodium sulfate or cysteine hydrochloride is added into the vial through a syringe to render the inside of the vial an absolutely anaerobic condition environment.

According to the method for decomposition of the present invention, it is recognized that the PHA sheet is decomposed within 30 days after the start of the cultivation.

When an organic substance such as tryptone or glucose, or an organic acid salt such as an acetate is caused to coexist with the components in the vial, the decomposition can be promoted; however, a reason therefor is unclear.

### Examples

Hereinafter, the present invention will be specifically described by way of examples. However, the technical scope of the invention is not limited by these examples.

[Enrichment of Microbial Community for Decomposition, Using Polyhydroxyalkanoic Acid Powder]

### (PHBH Powder Synthesis Method)

For culture and production, strain KNK-631 was used.

The composition of a seed culture medium was set as follows: 1 w/v% meat-extract, 1 w/v% Bacto-tryptone, 0.2 w/v% yeast-extract, 0.9 w/v% Na₂HPO₄·12H₂O, and 0.15 w/v% KH₂PO₄ (pH: 6.8).

The composition of a preculture medium was set as follows: 1.1 w/v% Na₂HPO₄·12H₂O, 0.19 w/v% KH₂PO₄, 1.29 w/v% (NH₄)₂SO₄, 0.1 w/v% MgSO₄·7H₂O, a 0.5 v/v% solution of trace metal salts (solution obtained by dissolving, into 0.1 N hydrochloric acid, 1.6 w/v% FeCl₃·6H₂O, 1 w/v% CaCl₂·2H₂O, 0.02 w/v% CoCl₂·6H₂O, 0.016 w/v% CuSO₄·5H₂O, and 0.012 w/v% NiCl₂·6H₂O). As a carbon source, a palm kernel oil was added thereto in a concentration of 10 g/L at a time.

The composition of a medium for producing a polyester resin was set as follows: 0.385 w/v% Na₂HPO₄·12H₂O, 0.067 w/v% KH₂PO₄, 0.291 w/v% (NH₄)₂SO₄, 0.1 w/v% MgSO₄·7H₂O, a 0.5 v/v% solution of trace metal salts (solution obtained by dissolving, into 0.1 N hydrochloric acid, 1.6 w/v% FeCl₃·6H₂O, 1 w/v% CaCl₂·2H₂O, 0.02 w/v% CoCl₂·6H₂O, 0.016 w/v% CuSO₄·5H₂O, and 0.012 w/v% NiCl₂·6H₂O), and 0.05 w/v% BIOSPUREX 200K (antifoaming agent, manufactured by Cognis Japan Ltd.).

Initially, a glycerol stock (50 µL) of strain KNK-631 was inoculated into the seed culture medium (10 mL) and cultivated for 24 hours to perform seed culture. The preculture medium (1.8 L) was inoculated with 1.0 v/v% of the seed culture broth in a 3-L jar fermenter (MDL-300 model, manufactured by B.E. MARUBISHI Co., Ltd.). The cultivation was performed for 28 hours under the conditions of a cultivation temperature of 33°C, a stirring rate of 500 rpm, a ventilation volume of 1.8 L/min, and a pH controlled between 6.7 and 6.8, to perform the preculture. The pH was controlled using a 14% aqueous solution of ammonium hydroxide.

Next, the medium for producing a polyester resin (6L) was inoculated with 1.0 v/v% of the preculture broth in a 10-L jar fermenter (MDS-1000 model, manufactured by B.E. MARUBISHI Co., Ltd.). The cultivation was performed under the conditions of a cultivation temperature of 28°C, a stirring rate of 400 rpm, a ventilation volume of 6.0 L/min, and a pH controlled between 6.7 and 6.8. The pH was controlled using a 14% aqueous solution of ammonium hydroxide. As a carbon source, palm oil was used. The cultivation was performed for 64 hours. After the cultivation, the cells were collected by centrifugation, washed with methanol, freeze-dried, and weighed to determine the dry cell weight.

To the obtained dry cells (1 g) was added chloroform (100 ml). The mixture was stirred at room temperature for one whole day and night, and the polyester resin in the cells was extracted. The extracted solution was filtered to remove cell residues, and concentrated in an evaporator to a total volume of about 30 ml. To the concentrated solution was gradually added hexane (90 ml), and the mixture was left to stand for one hour with slow stirring. The polyester resin thus precipitated was filtered off, and vacuum-dried at 50°C for three hours to yield a dry polyester resin. The 3HH composition of the resultant polyester resin was determined by gas chromatography as follows.

To 20 mg of the dried polyester resin were added 2 mL of a sulfuric acid/methanol mixed liquid (15 : 85) and 2 mL of chloroform, and the reactor was airtightly stopped. The reactor was heated at 100°C for 140 minutes to yield a methyl ester of a decomposition product of the polyester resin. The reactor was cooled, and then thereto was bit by bit added 1.5 g of sodium hydrogencarbonate to neutralize the reaction system. The system was allowed to stand still until the generation of carbon dioxide gas was stopped. Thereto was added 4 mL of diisopropyl ether, and the components were sufficiently mixed with each other, and then the system was subjected to centrifugation. The monomer unit composition of the polyester resin decomposition product in the supernatant was determined by capillary gas chromatography.

The gas chromatograph was GC-17A manufactured by SHIMADZU CORPORATION, and the capillary column was NEUTRA BOND-1 manufactured by GL Sciences Inc. (column length: 25 m, column internal diameter: 0.25 mm, and liquid membrane thickness: 0.4 µm). As a carrier gas, He was used. The inlet pressure of the column was set to 100 kPa, and 1 µL of a sample was injected into the column. For temperature conditions, the temperature was raised from a starting temperature of 100°C to 200°C at a rate of 8°C/min, and further raised from 200°C to 290°C at a rate of 30°C/min. As a result of the analysis under the above-mentioned conditions, the resultant polyester resin was a PHBH. The proportion of 3-hydroxybutyrate (3HB) in the PHBH was 89 mol%, and the proportion of 3-hydroxyhexanoate (3HH) was 11 mol%.

After the cultivation, a powder of the PHBH was yielded from the culture broth by the method described in WO 2010/067543. The weight-average molecular weight measured by the GPC was 650000.

### (Synthetic Wastewater Preparation Example 1)

Approximately 800 mL of distilled water was put into a 1-L volume beaker. While the water was stirred with a stirrer, thereto were added acetic acid, sodium acetate, potassium dihydrogenphosphate, potassium hydrogencarbonate, ammonium chloride, sodium chloride, magnesium chloride hexahydrate, cysteine hydrochloride monohydrate, a trace elements solution, and a vitamin solution. These components were then mixed with one another. Furthermore, an ultrasonic homogenizer was used to disperse the PHBH powder suspended in approximately 100 mL of distilled water, and then the resultant was added to the above-mentioned aqueous solution. Distilled water was used to increase up the volume of the present liquid to 1 L to prepare a synthetic wastewater a in which the carbon concentration ratio between the PHBH and the acetate (PHBH/acetate) was 1/9. The synthetic waste liquid a was prepared so as to adjust the total carbon concentration to 8,000 mg/L. The composition proportion of each of the compounds in the synthetic waste liquid a is shown in Table 1.

| [Table 1] | | | | |
|---|---|---|---|---|
| | | Synthetic wastewater a | Synthetic wastewater b | Synthetic wastewater c |
| Carbon concentration ratio of PHBH to acetate | | 1:9 | 2 : 8 | 3:7 |
| | Acetic acid | 14.4 g/L | 12.8 g/L | 11.2 g/L |
| | Sodium acetate | 4.91 g/L | 4.37 g/L | 3.82 g/L |
| | PHBH powder | 1.43 g/L | 2.85 g/L | 4.29 g/L |
| Composition ratio of each of compounds | Potassium dihydrogenphosphate | 0.30 g/L | | |
| | Potassium hydrogencarbonate | 4.00 g/L | | |
| | Ammonium chloride | 1.00 g/L | | |
| | Sodium chloride | 0.60 g/L | | |
| | Magnesium chloride hexahydrate | 0.82 g/L | | |
| | Cysteine hydrochloride monohydrate | 0.10 g/L | | |
| | Trace element solution | 10.00 ml/L | | |
| | Vitamin solution | 10.00 ml/L | | |

### (Synthetic Wastewater Preparation Examples 2 and 3)

In accordance with the method of Synthetic Wastewater Example 1, Synthetic wastewaters b and c were prepared, using a carbon concentration ratio shown in Table 1 between the PHBH and the acetate, and the composition ratio of each of the compounds.

### (Method for Measuring CODcr Removal Rate and Method for Calculating PHBH Decomposition Rate)

The CODcr (chemical oxygen demand: index of organic substance amount) in each of the synthetic waste liquid and the in-tank liquid of the reactor was measured, as the oxygen amount required when the organic substances in the sample were oxidized with potassium dichromate, using a COD reagent HR (Hach).

A value obtained by dividing the CODcr of the in-tank liquid of the reactor by the CODcr of the synthetic waste liquid was subtracted from a value of 1. The numerical value obtained by representing the obtained value in the unit of percent was defined as the CODcr removal ratio.

The water-soluble CODcr of the in-tank liquid of the reactor and the synthetic wastewater, and the total CODcr containing insoluble components were measured, respectively. From a difference therebetween, the CODcr of only the insoluble components was calculated. From this insoluble CODcr, the insoluble CODcr removal ratio was calculated. The resultant was defined as the PHBH decomposition ratio.

### (Method for Measuring Biogas Generation Velocity)

The biogas generated from the reactor was collected into a plastic gas-holder. At intervals of 24 hours, the amount of biogas generated was measured. The measured value was converted to a biogas generation amount per hour. The resultant numerical value was defined as the biogas generation velocity.

### [Enrichment of Microbial Community]

### (Example 1)

Anaerobic digest tank sludge containing a microorganism group shown in Table 2 was used as seed sludge. This was immersed in a thermostat water tank in which a completely stirred tank reactor having an actual volume of 1 L was adjusted to a temperature of 37°C (actual temperature: 36 to 38°C). This reactor was anaerobically operated. The emulsified synthetic wastewater a was continuously supplied to the completely stirred tank reactor.

Continuous cultivation was performed in a chemostat-manner in which the in-tank liquid was discharged at a rate equivalent to the synthetic wastewater supplying rate. Through this continuous cultivation, from microorganisms living in the seed sludge, a microbial community contributing to the decomposition of PHBH and the production of methane gas was enriched.

In the continuous cultivation, the synthetic wastewater a was supplied at a rate of a dilution rate of 0.025 d⁻¹, and the cultivation was continued over a term three times longer than the hydraulic retention time (40 d in this case). After the cultivation reached to a steady state, the treating performance of the reactor was evaluated through the CODcr removal ratio and the biogas generation velocity. The results are shown in Table 3.

**[Table 2]**

| Phylum | The number (proportion) of clones |
|---|---|
| Class | |
| ***Acidobacteria*** | **4.2%** |
| *Acidobacteria* | 4.2% |
| ***Firmicutes*** | **64.7%** |
| *Clostridia* | 31.3% |
| *Bacilli* | 33.4% |
| **Unclassified** | **14.6%** |
| **Total** | 83.5% |
| **Others** | 16.5% |

**[Table 3]**

| | Synthetic wastewater | Dilution rate (d⁻¹) | CODcr removal ratio (%) | Biogas generation velocity (ml/h) |
|---|---|---|---|---|
| Example 1 | a | 0.025 | 94.4 | 3.9 |
| Example 2 | b | 0.025 | 92.4 | 3.5 |
| Example 3 | b | 0.050 | 91.7 | 8.4 |
| Example 4 | c | 0.025 | 93.7 | 4.1 |
| Example 5 | c | 0.050 | 87.7 | 7.6 |

| | | | | |
|---|---|---|---|---|
| Dilution rate: "dilution rate D in continuous cultivation system" = "wastewater supply volume F per day"/"actual volume V" | | | | |

Ten percent of the organic carbon contained in this synthetic wastewater a originated from the PHBH. The supplied PHBH was decomposed in a proportion of about 44% to be made into inorganic substances.

### (Examples 2 to 5)

In the same manner as in Example 1 except that the synthetic wastewater and the dilution rate shown in Table 3 were used, the PHBH powder was treated, and the treating performance of the reactor in a steady state was evaluated through the CODcr removal ratio and the biogas generation velocity. The results are shown in Table 3.

Under the condition that the synthetic wastewater b was supplied in which the carbon concentration ratio between the PHBH and the acetate was 2/8, the CODcr removal ratio was 92.4% at a dilution rate of 0.025 d⁻¹ (Example 2), and was 91.7% at a dilution rate of 0.05 d⁻¹ (Example 3). Twenty percent of the organic carbon contained in this synthetic wastewater b originated from the PHBH. In the examples, approximately 62% by weight and approximately 58% by weight of the supplied PHBH were decomposed, respectively, to be made into inorganic substances.

Under the condition that the synthetic wastewater c was supplied in which the carbon concentration ratio between the PHBH and the acetate was 3/7, the CODcr removal ratio was 93.7% at a dilution rate of 0.025 d⁻¹ (Example 4), and was 87.7% at a dilution rate of 0.05 d⁻¹ (Example 5). Thirty percent of the organic carbon contained in this synthetic wastewater c originated from the PHBH. In the examples, approximately 79% by weight and approximately 59% by weight of the supplied PHBH were decomposed, respectively, to be made into inorganic substances.

Independently of the kind of the synthetic wastewater, the biogas generation velocity was approximately 4 mL/h at a dilution rate of 0.025 d⁻¹, and was approximately 8 mL/h at a dilution rate of 0.05 d⁻¹ to be substantially constant. Under all the reactor operating conditions, the decomposition of about 40 to 79% by weight of the PHBH powder was achieved. Thus, it is considered that microorganism groups for decomposing the PHBH anaerobically to produce methane were enriched in all the reactors.

### [Anaerobic Biological Decomposability Test of Polyhydroxyalkanoic Acid Sheet]

In order to evaluate the PHBH-sheet decomposing performance of the microorganism group enriched in the reactor in each of Examples 2 to 5, a decomposition test of a PHBH sheet was made in an anaerobic environment.

### (Method for Producing PHBH Sheet)

A co-rotating intermeshing type twin-screw extruder (TEM-26SS, manufactured by Toshiba Machine Co., Ltd.) was used to melt and knead a PHBH powder (3HH proportion: 11 mol%) at a set temperature of 100 to 140°C (outlet resin temperature: 160°C) and a screw rotation number of 100 rpm. From the dice, the extruded powder was taken out in the form of a strand, and the strand was cut into the form of pellets.

Subsequently, the resultant pellets were sufficiently dried at 60°C. Thereafter, extrusion was performed using a single-screw extruder ("20C200 model" Laboplast mill, manufactured by Toyo Seiki Seisaku-sho, Ltd.) to which a T-shaped dice having a lateral width of 150 mm and a lip width of 0.25 mm was fitted, at a shaping temperature of 140 to 170°C and a screw rotation number of 50 rpm, and a sheet was pulled out through cooling rolls having an adjusted temperature of 55°C to yield a T die sheet having a width of about 110 mm and a thickness of about 50 µm.

### (Evaluation of Decomposability)

From the medium subjected to the decomposing treatment, the PHBH sheet was taken out, and the state of the decomposition of the PHBH sheet was evaluated in accordance with four stages based on the following criterion:
-: The sheet is not decomposed (the shape of the sheet is not changed).
+: The sheet is broken (the sheet is decomposed to be cut or perforated so that the sheet is partially broken, but the original shape of the sheet, which is rectangular, is kept).
++: The sheet collapses (the original shape of the sheet is not recognized, but the wreck of the decomposed sheet is observed).
+++: The sheet disappears (neither the original shape of the sheet nor the wreck of the sheet is seen in the cultured liquid).

### (Example 6)

Into a 100-mL-volume glass vial were put 40 mL of a mineral salt medium having composition shown in Table 4, and a single PHBH sheet having a thickness of 50 µm and cut into a size of 1 cm x 4 cm. Oxygen in the medium was then substituted with a mixed gas of N₂ and CO₂ (ratio of N₂ to CO₂ = 80/20). Thereafter, the vial was airtightly stopped with a butyl rubber stopper, and then sodium sulfate and cysteine hydrochloride were added thereto through a syringe to make the inside into an absolutely anaerobic environment.

**[Table 4]**

| Component | Final concentration |
|---|---|
| Potassium dihydrogenphosphate | 0.30 g/L |
| Potassium hydrogencarbonate | 4.00 g/L |
| Ammonium chloride | 1.00 g/L |
| Sodium chloride | 0.60 g/L |
| Magnesium chloride hexahydrate | 0.82 g/L |
| Resazurin | 1.0 mg/L |
| Trace elements solution | 10.00 ml/L |
| Vitamin solution | 10.00 ml/L |

The in-tank liquid of the reactor in Example 2 was added to this vial through a 10-mL syringe, and the microorganisms therein were cultivated in a batch manner at 37°C while the liquid was shaken. During the cultivation, the decomposability of the sheet was timely evaluated. The results are shown in Table 5. From the 9^{th} day after the start of the cultivation to the 27^{th} day, the decomposition of the PHBH sheet was recognized.

**[Table 5]**

| | Used in-tank liquid | Culture period (d) | | | | |
|---|---|---|---|---|---|---|
| | | 9 | 12 | 15 | 21 | 27 |
| Example 6 | Example 2 | - | - | + | ++ | ++ |
| Example 7 | Example 3 | - | - | - | - | ++ |
| Example 8 | Example 4 | - | - | - | - | ++ |
| Example 9 | Example 5 | + | + | + | ++ | ++ |
| Comparative Example 1 | - | - | - | - | - | - |

### (Examples 7 to 9)

Cultivation was performed and the decomposability was evaluated in the same manner as in Example 6 except that the in-tank liquid of the reactor shown in Table 5 was used. The results are shown in Table 5.

Independently of the reactor operation conditions for the used in-tank liquids, from the 21^{th} day after the start of the culture to the 27^{th} day, the PHBH sheets collapsed remarkably.

It was understood that the microbial community enriched in each of the reactors has the PHBH-sheet decomposing performance.

### (Comparative Example 1)

In the same manner as in Example 6 except that an in-tank liquid of an anaerobic digest tank containing the microbial community shown in Table 2, which was seed sludge in Example 1, was used, the decomposability of the PHBH sheet was evaluated. The results are shown in Table 5.

### [Anaerobic Biological Decomposability Test of Acetate-Added Polyhydroxyalkanoic Acid Sheet]

An acetate was contained in the synthetic waste liquid supplied to the reactor in each of Examples 1 to 5. Evaluations were made for the PHBH-sheet decomposing capability based on the coexistence with an acetate.

### (Example 10)

Cultivation was performed and the decomposability was evaluated in the same manner as in Example 6 except that 6.8 g/L of an acetate was further added to the vial, and in this state the culture was performed in a batch manner. The results are shown in Table 6.

It was understood that the addition of an acetate gives the better PHBH-sheet decomposing performance than the addition of no acetate.

**[Table 6]**

| | Used in-tank liquid | Culture period (d) | | | | | |
|---|---|---|---|---|---|---|---|
| | | 3 | 4 | 5 | 6 | 7 | 21 |
| Example 10 | Example 2 | - | +++ | +++ | +++ | +++ | +++ |
| Example 11 | Example 3 | - | - | ++ | +++ | +++ | +++ |
| Example 12 | Example 4 | +++ | +++ | +++ | +++ | +++ | +++ |
| Example 13 | Example 5 | - | - | - | - | + | ++ |

### (Examples 11 to 13)

Cultivation was performed and the decomposability was evaluated in the same manner as in Example 10 except that the in-tank liquid of the reactor shown in Table 6 was used. The results are shown in Table 6.

In any one of these cases, it was understood that the addition of the acetate gives the better PHBH-sheet decomposing performance than the addition of no acetate.

### [Anaerobic Biological Decomposability Test of Organic Additive-Added Polyhydroxyalkanoic Acid Sheet]

When a PHBH sheet is used for a garbage bag, it is necessary to use methane fermentation to treat kitchen wastes in the state of being put in the bag. Thus, the decomposability of a PHBH sheet was evaluated in the coexistence of an organic substance.

### (Example 14)

Culture was performed and the decomposability was evaluated in the same manner as in Example 6 except that tryptone, which is a hydrolyzate of a protein, was further added to the vial to give a concentration of 10 g/L, and in this state the culture was performed in a batch manner. The results are shown in Table 7.

It was understood that even under conditions of the addition of the hydrolyzate of the protein, the decomposability is not lowered so that the conditions give the better PHBH sheet-decomposing performance than under conditions that such an additive is not added.

**[Table 7]**

| | Used in-tank liquid | Culture period (d) | | | | | |
|---|---|---|---|---|---|---|---|
| | | 2 | 3 | 4 | 8 | 9 | 12 |
| Example 14 | Example 2 | - | - | - | + | +++ | +++ |
| Example 15 | Example 3 | - | ++ | +++ | +++ | +++ | +++ |
| Example 16 | Example 4 | - | - | - | - | - | ++ |
| Example 17 | Example 5 | +++ | +++ | +++ | +++ | +++ | +++ |

### (Examples 15 to 17)

Culture was performed and the decomposability was evaluated in the same manner as in Example 14 except that the in-tank liquid of the reactor shown in Table 7 was used. The results are shown in Table 7.

In any one of these cases, it was understood that even under conditions of the addition of the hydrolyzate of the protein, the decomposability is not lowered so that the conditions give the better PHBH sheet-decomposing performance than under conditions that such an additive is not added.

### (Example 18)

Batch culture was performed in the same manner as in Example 16 except that 5 g/L of glucose together with 10 g/L of tryptone was further added into the vial, and in this state, the batch culture was performed. As a result, on the 5^{th} day after the start of the batch culture, the sheet collapsed. It was understood from this matter that even under conditions that an organic substance originating from a protein coexists with an organic substance originating from a carbohydrate, the microbial community enriched in the reactor shows the PHBH-sheet decomposing performance.

### [Observation of Microorganisms Growing in Each Reactor through Microscope]

### (Example 19)

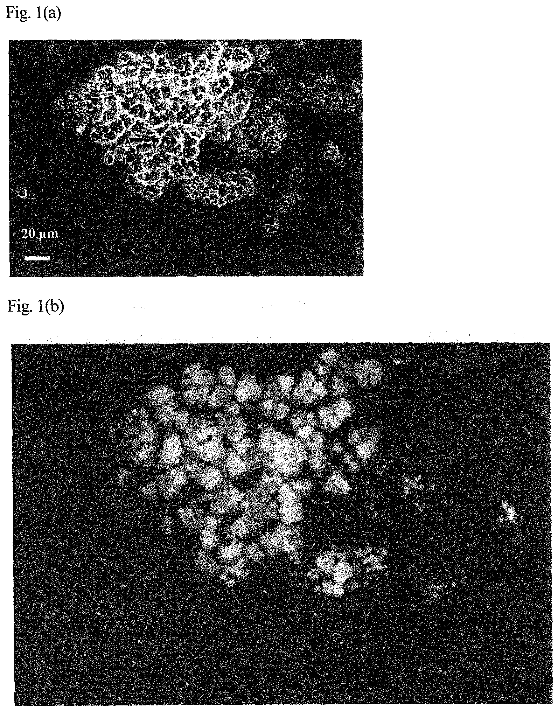

The in-tank liquid of the reactor in Example 3 was collected, and an epifluorescence microscope (BX-41, manufactured by Olympus Corporation) was used to observe cells present in the in-tank liquid. The results are shown in Fig. 1.

In the reactor tank, cells forming aggregates were dominant. Besides, cells of various microorganisms including micrococcus, bacilli, and single bacilli were observed. A coenzyme F₄₂₀ peculiar for hydrogen-utilizing methane-producing archaeon was detected through fluorescence observation, so that it was observed that the cells, which formed the aggregates, emitted pale fluorescence.

It can be considered that the cells, which formed the aggregates and emitted autofluorescence of a coenzyme F₄₂₀, were methane-producing archaeons belonging to the genus Methanosarcina, which utilize acetic acid, hydrogen and carbon dioxide to produce methane. Simultaneously, single-rod-shaped cells that show autofluorescence were also observed. This matter suggests the presence of hydrogen-utilizing methane-producing archaeons belonging to other than the genus Methanosarcina.

Cells of various microorganisms composed of micrococcus, bacilli, and single bacilli showing no autofluorescence are bacteria which decompose PHBHs directly or indirectly. These bacteria decompose PHBHs to produce acetic acid, hydrogen and carbon dioxide, which are substrates for methane-producing archaeon.

### (Examples 20 to 23)

For the in-tank liquid of the reactor of each of Examples 1,2,4 and 5, an observation was made through the microscope in the same manner as in Example 19. As a result, independently of a difference in carbon concentration ratio between the PHBH and the acetate in the synthetic waste liquid to be supplied and a difference in dilution rate between these examples, the same results as in Example 19 were obtained.

### [Construction of Clone Library of 16S rRNA Genes]

### (Example 24)

A DNA extracting kit ISOIL for beads beating (manufactured by Nippon Gene Co., Ltd.) was used to extract DNA of the microorganisms growing in the in-tank liquid of the reactor in Example 3. The resultant DNA was used as a template for amplification of 16S rRNA genes of the bacteria included in the microorganisms by a PCR method using a Go Taq Green Master Mix (manufactured by Promega). PCR primers used were EUB338 mixF and 1490R (5'-GGTTACCTTGTTACGACTT-3').

As a result, DNA fragments of 1100 base pairs were amplified as a PCR product. The resultant DNA fragments were purified using a MinElute PCR purification kit (manufactured by Qiagen). A TOPO TA cloning kit (manufactured by Life Technologies) was used to link the purified DNA fragments to vector plasmids pCR 2.1-TOPO, and then the plasmids were introduced into Escherichia coli to construct a gene library composed of clones of the 16S rRNA genes of the bacteria in the reactor, the number of the clones being about 50.

### (Examples 25 to 27)

In the same manner as in Example 24 except that the in-tank liquid of the reactor in each of Examples 2,4 and 5 was used, a gene library composed of clones of the 16S rRNA genes, the number of the clones being about 50, was constructed from the reactor.

### [Phylogenetic Systematics of Microorganisms Growing in Each Reactor on Basis of Base Sequences of 16S rRNA Genes]

### (Example 28)

The base sequences of 16S rRNA gene fragment inserted into the plasmids contained in the transformant obtained in Example 24 were determined using a sequence primer 907R. The determination of the base sequences was made in Dragon Genomics Center of Takara Bio Inc. The resultant base sequences were subjected to homology search by using the BLAST program of NCBI (National Center for Biotechnology Information) (http://www.ncbi.nlm.nih.gov/). Thereafter, the ARB program (http://www.arb-home.de/) of Technische Universität München was used to perform an alignment of 16S rRNA to make a phylogenetic analysis. Groups each having base pairs having a sequence homology of 97% or more therebetween, out of approximately 500 base pairs of the decoded 16 rRNA gene sequences, which included those of a V3-V4 region, were each rendered a group as the same operational taxonomy units (OTUs). For the classification of a representative sequence of each OTU, a gene sequence obtained by the homology search according to the BLAST program and having a highest homology was compared with sequences in a data base SILVA ver. 117 of the SILVA ribosomal database project (http://www.arb-silva.de) of Max Planck Institute, and then a classification belonging to the representative sequence was determined. Actually, from the 16S rRNA gene sequences originating from each of the reactors, 44 OTUs were obtained. Next, a final genealogical tree was produced by the neighbor joining method (NJ method) and the maximum parsimony method (MP method) with use of a 16S rRNA gene sequence of a species having a highest homology to the base sequence of the representative clone of each OTU.

Table 8 shows results obtained by classifying the 16S rRNA genes originating from the reactor at a phylum level and a class level.

**[Table 8]**

| | Example 28 | Example 29 | Example 30 | Example 31 |
|---|---|---|---|---|
| Used reactor | Example 3 | Example 2 | Example 5 | Example 4 |
| Carbon concentration ratio of PHBH to acetate | 2:8 | | 3:7 | |
| Dilution rate (d⁻¹) | 0.05 | 0.025 | 0.05 | 0.025 |
| Phylum | The number (prop ortion) of clones | | The number (pro portion) of clones | |
| Class | | | | |
| ***Acidobacteria*** | 2 (4.7%) | 0 (0%) | 0 (0%) | 0 (0%) |
| "Subgroup 3" | 1 | 0 | 0 | 0 |
| Unclassified | 1 | 0 | 0 | 0 |
| ***Bacteroidetes*** | 7 (16.3%) | 0 (0%) | 6 (0%) | 3 (6.3%) |
| *Bacteroidia* | 0 | 0 | 0 | 1 |
| *Sphingobacteriia* | 7 | 0 | 6 | 2 |
| ***Chloroflexi*** | 0 (0%) | 1 (2.1%) | 1 (2.1%) | 0 (0%) |
| *Anaerolineae* | 0 | 1 | 1 | 0 |
| ***Firmicutes*** | 30 (69.8%) | 35 (72.9%) | 37 (78.7%) | 35 (72.9%) |
| *Clostridia* | 25 | 19 | 23 | 31 |
| *Bacilli* | 0 | 14 | 0 | 0 |
| *Negativicutes* | 3 | 0 | 12 | 0 |
| *Erysipelotrichia* | 0 | 0 | 2 | 0 |
| "OPB54" | 2 | 2 | 0 | 4 |
| ***Nitrospirae*** | 0 (0%) | 1 (2.1%) | 0 (0%) | 0 (0%) |
| *Nitrospira* | 0 | 1 | 0 | 0 |
| ***Proteobacteria*** | 3 (7.0%) | 11(22.9) | 0 (0%) | 7 (14.6%) |
| *Betaproteobacteria* | 3 | 6 | 0 | 7 |
| | 0 | 5 | 0 | 0 |
| *Gammaproteobacteria* | | | | |
| ***Synergistetes*** | 0 (0%) | 0 (0%) | 1 (2.1%) | 0 (0%) |
| *Synergistia* | 0 | 0 | 1 | 0 |
| ***Thermotogae*** | 1 (2.3%) | 0 (0%) | 0 (0%) | 0 (0%) |
| *Thermotogae* | 1 | 0 | 0 | 0 |
| **Unclassified** | 0 (0%) | 0 (0%) | 2 (4.3%) | 3 (6.3%) |
| Total | 43 | 48 | 47 | 48 |

### (Examples 29 to 31)

In the same manner as in Example 28 except that the in-tank liquid of the reactor in each of Examples 2, 4 and 5 was used, the 16S rRNA genes originating from the reactor were classified at a phylum level and a class level. The results are shown in Table 8.

It was made clear that in accordance with the conditions for operating the reactors, the constitution of bacteria was varied, and the bacteria were distributed in total to nine phyla (including an unclassified phylum-level group). In particular, clones classified into the phylum Firmicutes were remarkably detected with a high frequency regardless of the reactor operating conditions. For the respective 16S rRNA genes obtained from the following reactors, 69.8%, 72.9%, 78.7% and 72.9% of the genes were classified, respectively, into the phylum Firmicutes: the reactor in which the synthetic wastewater having a carbon concentration ratio of the PHBH to the acetate of 2/8 was supplied and operated at a dilution ratio of 0.05 d⁻¹ (Example 3); the reactor in which the same wastewater was supplied and operated at a dilution ratio of 0.025 d⁻¹ (Example 2); the reactor in which the synthetic wastewater having a carbon concentration ratio of the PHBH to the acetate of 3/7 was supplied and operated at a dilution ratio of 0.05 d⁻¹ (Example 5); and the reactor in which the same wastewater was supplied and operated at a dilution ratio of 0.025 d⁻¹ (Example 4). Furthermore, the clones of each phylum were classified at a class level. The clones classified into the class Clostridia were detected in a large quantity. This is a characteristic constitution of bacterial community in reactors for decomposing PHBHs. It can be considered that bacteria classified into the phylum Firmicutes, in particular, bacteria classified into the class Clostridia play a main role of decomposing PHBHs.

### (Example 32)

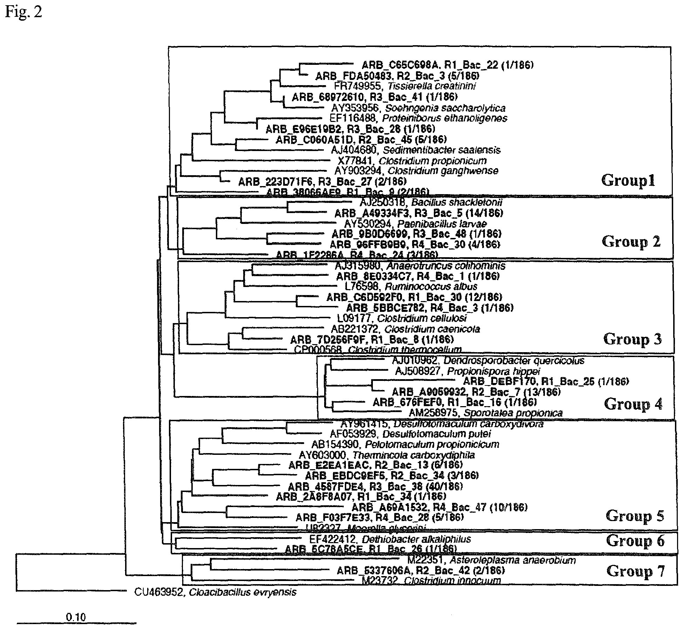

Out of the 16S rRNA genes originating from the reactor in Example 3, genes classified into the phylum Firmicutes were further classified in detail. A phylogenetic tree thereof was prepared to demonstrate that the 16S rRNA genes classified into the phylum Firmicutes were further classified into 7 groups (Fig. 2 and Table 9).

### (Examples 33 to 35)

For each of Examples 2, 4 and 5, in the same manner as in Example 32, out of the 16S rRNA genes originating from the reactor in the example, genes classified into the phylum Firmicutes were further classified in detail. The results are shown in Fig. 2 and Table 9.

**[Table 9]**

| | Example 32 | Example 33 | Example 34 | Example 35 |
|---|---|---|---|---|
| Reactor number | Example 3 | Example 2 | Example 5 | Example 4 |
| Carbon concentration ratio of PHBH to acetate | 2:8 | | 3:7 | |
| Dilution ratio (d⁻¹) | 0.05 | 0.025 | 0.05 | 0.025 |
| Classification | The number (proportion) of clones | | The number (proportion) of clones | |
| Group 1 | | | | |
| Class *Clostridia* No. XI | | 6 4 7 | | 0 |
| Unclassified family, Family *Clostridiaceae* | (20%) | (11.4%) | (18.9%) | (0%) |
| Family *Calidicoprobacteraceae* | | | | |
| Group 2 | | | | |
| Class *Clostridia* No. XII, Unclassified family, | 2 | 16 | 0 | 4 |
| | (6.7%) | (45.7%) | (0%) | (11.4%) |
| Clone OPB54 related species | | | | |
| Group 3 | | | 8 0 5 | 2 |
| (Family *Ruminococcaceae*) | (26.7%) | (0%) | (13.5%) | (5.7%) |
| Group 4 | 3 | 0 | 12 | 0 |
| (Family *Veillonellaceae*) | (10.0%) | (0%) | (32.4%) | (0%) |
| Group 5 | | | | |
| Family *Peptococcaceae* | 10 | 15 | 11 | 29 |
| Family *Thermoanaerobacteraceae* | (33.3%) | (42.9%) | (29.7%) | (82.9%) |
| Group 6 | 1 | 0 | 0 | 0 |
| (Class *Clostridia* No. XIV, Unclassified family) | (3.3%) | (0%) | (0%) | (0%) |
| Group 7 | 0 | 0 | 2 | 0 |
| (Family *Erysipelotrichaceae*) | (0%) | (0%) | (5.4%) | (0%) |
| Total | 30 | 35 | 37 | 35 |

| | | | | |
|---|---|---|---|---|
| * Unclassified family: this is a family-level classified group present in the class Clostridia, but detailed characteristics thereof are undetermined (Bergey's Manual of Systematic Bacteriology second ed., Volume three "The Firmicutes" edited by P. de Vos et al., Springer (2009)). * Clone OBP54: this is a clone detected as a 16S rRNA gene clone from an environment sample, but a detailed classification is undetermined (P. Hugenholtz et. al., J. Bacteriol.,180, 266 (1998)). | | | | |

Fig. 2 shows a phylogenetic tree of the 16S rRNA gene clones detected in the reactors within the phylum Firmicutes. The clones obtained in Examples 32 to 35 are represented by boldfaced types. Inside each parenthesis, the following are represented: the number of the detected clones; and the total number of the clones (including clones other than the clones belonging to the class Firmicutes), i.e., 186. The length of each bar in the figure shows a 10% mismatch of the base sequences. As an outgroup, a bacterium Cloacibacillus evryensis belonging to the phylum Synergistetes was used.

Out of the seven groups, a group into which many clones were classified regardless of the reactor operation conditions was group 5, which is a group constituted by bacteria in the family Peptococcaceae and the family Thermoanaerobacteraceae. It can be considered that out of bacteria belonging to t the class Clostridia within the phylum Firmicutes, particularly, bacteria belonging to this group play a main role of decomposing PHBHs.

## Claims

1. A method for biologically decomposing a polyhydroxyalkanoic acid, the method comprising: subjecting a polyhydroxyalkanoic acid to decomposing treatment with a microbial community comprising a methane-producing archaeon in (A) below and a bacterium in (B) below:
(A) a methane-producing archaeon belonging to the genus Methanosarcina, or a single-rod-shaped hydrogen-utilizing methane-producing archaeon, and
(B) a bacterium which is classified into any one of the phylum Acidobacteria, the phylum Bacteroidetes, the phylum Chloroflexi, the phylum Firmicutes, the phylum Nitrospirae, the phylum Proteobacteria, the phylum Synergistetes, the phylum Thermotogae and an unclassified phylum, and is capable of decomposing the polyhydroxyalkanoic acid.

2. The method for decomposing a polyhydroxyalkanoic acid according to claim 1, wherein the archaeon in (A) comprises the methane-producing archaeon belonging to the genus Methanosarcina.

3. The method for decomposing a polyhydroxyalkanoic acid according to claim 1, wherein the methane-producing archaeon in (A) comprises both of the methane-producing archaeon belonging to the genus Methanosarcina and the single-rod-shaped hydrogen-utilizing methane-producing archaeon.

4. The method for decomposing a polyhydroxyalkanoic acid according to any one of claims 1 to 3, wherein the bacterium in (B) comprises a bacterium classified into the phylum Firmicutes.

5. The method for decomposing a polyhydroxyalkanoic acid according to claim 4, wherein a proportion of the bacterium classified into the phylum Firmicutes in a whole of the microbial community is from 50 to 95% both inclusive in terms of a proportion of clones of a 16S rRNA gene.

6. The method for decomposing a polyhydroxyalkanoic acid according to claim 4 or 5, wherein the bacterium classified into the phylum Firmicutes comprises a bacterium classified into the family Peptococcaceae or the family Thermoanearobacteraceae in a total proportion of 20 to 95% both inclusive in terms of the proportion of the clones of the 16S rRNA gene.

7. The method for decomposing a polyhydroxyalkanoic acid according to any one of claims 1 to 6, wherein the polyhydroxyallcanoic acid is subjected to the decomposing treatment under an anaerobic condition.

8. The method for decomposing a polyhydroxyallcanoic acid according to any one of claims 1 to 7, wherein the polyhydroxyalkanoic acid is subjected to the decomposing treatment under a condition of a temperature from 20 to 65°C.

9. The method for decomposing a polyhydroxyalkanoic acid according to any one of claims 1 to 8, wherein the polyhydroxyalkanoic acid is a polymer comprising, as a recurring unit structure, at least one of 3-hydroxybutyric acid and 3-hydroxyhexanoic acid.

10. The method for decomposing a polyhydroxyalkanoic acid according to any one of claims 1 to 8, wherein the polyhydroxyalkanoic acid is a polymer comprising, as recurring unit structures, 3-hydroxybutyric acid and 3-hydroxyhexanoic acid.

11. A microbial preparation for decomposing a polyhydroxyalkanoic acid and producing methane, the preparation comprising a methane-producing archaeon in (A) below and a bacterium in (B) below:
(A) a methane-producing archaeon belonging to the genus Methanosarcina, or a single-rod-shaped hydrogen-utilizing methane-producing archaeon, and
(B) a bacterium which is classified into any one of the phylum Acidobacteria, the phylum Bacteroidetes, the phylum Chloroflexi, the phylum Firmicutes, the phylum Nitrospirae, the phylum Proteobacteria, the phylum Synergistetes, the phylum Thermotogae, and an unclassified phylum, and is capable of decomposing the polyhydroxyalkanoic acid.
